# EUROPÄISCHE PATENTSCHRIFT

(11) **EP 3 615 118 B1**
(45) Veröffentlichungstag und Bekanntmachung des Hinweises auf die Patenterteilung: **26.04.2023**
(21) Anmeldenummer: 18712430.0
(22) Anmeldetag: 16.02.2018
(51) Int. Cl.: A61B 5/021, A61B 5/024, A61B 5/06, A61B 5/145, A61B 5/00, G06T 11/20, A61N 1/39, A61M 16/00, A61B 5/08

(54) **ANZEIGE ZUR AUSGABE VON INFORMATIONSGEHALTEN MEDIZINTECHNISCHER VORRICHTUNGEN**
DISPLAY FOR OUTPUTTING INFORMATION CONTENTS OF MEDICAL DEVICES
AFFICHAGE POUR LA PRÉSENTATION DE CONTENUS D'INFORMATIONS DE DISPOSITIFS MÉDICAUX

(30) Priorität: 27.04.2017 DE 102017004140; 09.08.2017 DE 102017007625
(43) Veröffentlichungstag der Anmeldung: 04.03.2020
(73) Patentinhaber: WEINMANN Emergency Medical Technology GmbH + Co. KG, 22525 Hamburg (DE)
(72) Erfinder: NEUHAUS, Christian, 22457 Hamburg (DE); SCOTTI, Norman, 25980 Sylt OT Tinnum (DE); DORDEVIC, Milos, 22305 Hamburg (DE)
(74) Vertreter: Klickow & Wetzel PartGmbB
(86) Internationale Anmeldenummer: PCT/DE2018/000040
(87) Internationale Veröffentlichungsnummer: WO 2018/196894

(56) Entgegenhaltungen:
- EP-A1- 0 872 255
- EP-A1- 1 859 830
- EP-A2- 1 834 622
- EP-A2- 3 101 882
- WO-A1-2016/018448
- WO-A1-2017/047595
- WO-A2-02/058619
- DE-B3-102011 122 905
- DE-U1- 20 315 975
- US-A- 5 007 420
- US-A- 5 735 799
- US-A1- 2006 173 501
- US-A1- 2009 141 593
- US-A1- 2013 314 522
- US-A1- 2015 105 687
- US-A1- 2015 138 205
- US-B2- 8 016 765
- HELBIG MEDIZINTECHNIK: "Helbig Katalog 2004/2005", INTERNET CITATION, 1 January 2004 (2004-01-01), XP002385041, Retrieved from the Internet: URL:http://www.helbig.de/Helbig-2004-05-lo w.pdf [retrieved on 2006-06-12]

## Beschreibung

Die Erfindung betrifft eine medizintechnische Vorrichtung mit einer Anzeige zur Ausgabe von Informationsgehalten wenigstens eines Parameters, Einstell- oder Messwertes innerhalb wenigstens eines Anzeigenbereiches.

Die Medizintechnik verfügt seit geraumer Zeit über verschiedene Geräte oder Vorrichtungen zur Unterstützung, Aufrechterhaltung oder Verbesserung verschiedener biologischer Funktionen von Menschen und allgemein von Säugetieren und Lebewesen. Ebenfalls verfügbar sind Vorrichtungen mit reanimierenden Aufgabenstellungen für insbesondere lebenswichtige biologische Funktionen oder Prozesse. Biologische Funktionen oder Prozesse in diesem Zusammenhang können beispielsweise die Ein- und Ausatmung oder die Herzfunktion sein.

Medizintechnische Geräte zur Unterstützung, Aufrechterhaltung oder Verbesserung der Ein- und Ausatmung können beispielsweise durch Beatmungsgeräte und Notfallbeatmungsgeräte realisiert sein. Ein anderer bekannter Gerätetyp sind Dialysemaschinen zur Verwirklichung von Blutreinigungsaufgaben. Bei der Reanimation von Herzfunktionen werden insbesondere Defibrillatoren eingesetzt.

Vor, während und gegebenenfalls auch nach der Verwendung von Geräten oder Vorrichtungen zur Unterstützung, Aufrechterhaltung oder Verbesserung verschiedener biologischer Funktionen werden eine Vielzahl von Parametern, Einstellwerten, Messwerten insbesondere physikalischer Natur benötigt und/oder gemessen und/oder erfasst beziehungsweise verarbeitet. Gleiches gilt für deren Ausgabe.

Der Ausgabe von Messwerten kommt eine besondere Bedeutung zu. Dies gilt in besonderem Maße für die richtige, korrekte und zielgerichtete Verwendung derartiger Vorrichtungen durch Bedienungspersonal, Ärzte oder im Zusammenspiel mehrerer Vorrichtungen. Aus diesem Grund werden insbesondere in Beatmungsgeräten und Defibrillatoren eine Vielzahl unterschiedlicher, vorzugsweise physikalischer Messwerte und/oder Funktionsparameter ermittelt, gemessen oder erfasst.

Je nach Typ und inhaltlichem Informationsgehalt der Parameter, Einstellwerte oder Messwerte und/oder deren Ausprägung in digitaler oder analoger Form ist es erforderlich, eine geeignete Darstellung mit gegebenenfalls vorangehender Aufbereitung bereitzustellen.

Zur Realisierung derartiger Ausgaben werden häufig analoge oder digitale Anzeigen, beispielsweise über Displays oder spezielle Anzeigeeinrichtungen verwendet, die einzelne und/oder Folgen von Mess-, Parameter-, Einstell- und sonstigen physikalischen Werten abbilden. Gleiches gilt für die Kombination mehrerer Werte oder Wertescharen, die auch in zwei- oder dreidimensionalen Anzeigen in Form von Wertefeldern dargestellt werden. Bekannt in diesem Zusammenhang sind auch graphische Ausgaben auf Displays in Form von Diagrammen, Balken oder Kurven.

Insbesondere bei Vorrichtung mit reanimierenden und/oder beatmenden Aufgabenstellungen für insbesondere lebenswichtige biologische Funktionen oder Prozesse ist es von lebenswichtiger Bedeutung, dass die Ausgabe der Informationsgehalte von Parametern, Einstellwerten oder Messwerten und/oder deren Ausprägung schnell, unzweifelhaft und auch unter Störeinflüssen absolut zuverlässig erfolgt und derart dargeboten wird, dass Bedienungspersonal den Informationsinhalt unzweifelhaft und korrekt aufnehmen kann.

Bei bekannten Ausgabeformen und -vorrichtungen sind die Informationsgehalte der Parameter, Einstellwerte oder Messwerte und/oder deren Ausprägung nicht immer zuverlässig ables- oder erfassbar. Eine Vielzahl von Fehlerquellen können additiv oder alternativ auftreten: Visuelle Fehlinterpretation, optische Verwechslung mehrerer gleichzeitig dargebotener Werte, zu langsame Aktualisierung auf dem Bildschirm oder Display, ungeeignete Abbildungsform digitaler oder analoger Werte oder Wertefolgen usw.

Aus der WO 2017/04 7595 A1 ist bereits die Verwendung einer tachometerartigen Anzeige im Bereich einer medizintechnischen Vorrichtung bekannt.

Weitere Anzeigen für medizintechnische Vorrichtungen werden in der WO 02/05 8619 A2, der US 5007 420 A sowie der WO 2016/018448 A1 beschrieben und/oder dargestellt.

Bei medizintechnischen Geräten ist die Verwendung von Anzeigen, die ähnlich zu einem Tachometer gestaltet sind, bereits bekannt. Entsprechende Realisierungen von derartigen Anzeigen werden beispielsweise in den Dokumenten EP 1 834 622 A2, US 2006/173501 A1, EP 1 859 830 A1, Helbig Medizintechnik "Helbig Katalog 2004/2005", Internet Citation, 1. Januar 2004 (2004-01-01), XP002385041, Gefunden im Internet: URL:http://www.helbig.de/Helbig-2004-05-low.pdf, DE203 15 975 U1, US 2015/138205 A1 beschrieben.

Es ist Aufgabe der Erfindung, die zuvor genannten Nachteile bei der Ausgabe der Informationsgehalte von Parametern, Einstellwerten oder Messwerten und/oder deren Ausprägung wenigstens zu reduzieren. Insbesondere ist es Aufgabe, in der speziellen Situation der Wiederbelebung mittels Defibrillatoren im Zusammenhang mit einer Beatmung die wichtigsten Parameter für den Anwender schnell ablesbar zu visualisieren, wobei die Genauigkeit in den Hintergrund tritt und das Einhalten von relevanten Grenzwerten erleichtert. Weiterhin ist es Aufgabe, eine leicht ablesbare Anzeige für Reanimationsgeräte oder Beatmungsgeräte, insbesondere für Notfallbeatmungsgeräte bereitzustellen.

Die Aufgabe wird durch die Merkmalskombination von Patentanspruch 1 gelöst.

Die nachfolgende Offenbarung sieht eine Anzeige in, für oder interagierend mit wenigstens einem Gerät beziehungsweise eine Vorrichtung vor, bestehend aus beispielsweise einem Defibrillator und/oder einem Beatmungsgerät und/oder einem Kombinationsgerät und/oder Monitor, welches über eine Messeinrichtung für diskrete, kontinuierliche und/oder einem oder mehrerer periodisch auftretender Signale mit Informationsgehalten hinsichtlich Parametern, Einstellwerten oder Messwerten und/oder deren Ausprägung verfügt. Vorgesehen ist, dass Informationsgehalte und/oder physikalische Größen als Einzelwerte in diskreter Form, fortlaufend, alternierend und/oder intermittierend ausgegeben werden. Messwerte und andere darzustellende Informationsgehalte können teilweise oder vollständig auch von einem anderen Gerät stammen und einmalig, intervallbehaftet oder kontinuierlich zum anzeigenden Gerät übermittelt werden.

Die Signale, Informationsgehalte und/oder auch Handlungsanweisungen für Bedienungspersonal werden als zumindest ein Hauptmerkmal auf einem Display in Form einer tachometerartigen Anzeige ausgegeben. Zusätzlich wird farblich kenntlich gemacht, ob die Grenzen für den angezeigten Parameter eingehalten werden. Zusätzlich kann der Wert numerisch angezeigt werden. Es werden zusätzlich eine Kurve oder Trenddarstellungen unter oder oberhalb der tachometerartigen Anzeige ausgegeben, beispielsweise um langsamere veränderliche Prozesse darzustellen. Im Weiteren können Alarme und andere Messwerte und Statusdaten dargestellt werden.

Im Besonderen kann es sich bei der tachometerartigen Anzeige um die abzubildende Triggerfrequenz eines Beatmungsgerätes handeln, in dessen Beatmungsmodus das Triggersignal auftreten kann. Die anzuzeigenden zulässigen Grenzwerte sind hierbei beispielsweise die Empfehlungen des ERC (European Resuscitation Council VZW) von 100-120/min. Die Kurve kann eine Atemwegsflowkurve oder eine ETCO2 Trendkurve sein.

Die nachfolgende Offenbarung erkennt, dass die zumindest teilweise Darstellung von Informationsgehalten von Parametern, Einstellwerten oder Messwerten und/oder deren Ausprägung in diskreter und/oder kontinuierlicher, alternierender, asymptotischer Weise durch wenigstens eine tachometerartige Anzeige erhebliche Vorteile bietet und insbesondere die Nachteile bekannter Anzeigen zumindest teilweise reduziert. Die tachometerartige Anzeige verwirklicht dabei beispielsweise eine Funktionsweise von Zeigerinstrumenten mit rotativem Zeiger wie sie in analogen Geschwindigkeitsmessern in Form von Tachometern in Fahrzeugen Verwendung finden. Eine entsprechende Anzeige ist auch in Notfallsituationen leicht und schnell ablesbar. Insbesondere ist daran gedacht, den Anzeigebereich in unterschiedlichen Farben zu gestalten. Beispielsweise wird ein Bereich mit zulässigen Werten in Grün dargestellt, kritische Werte hingegen in Rot.

Erfindungsgemäß erfolgt eine Verwendung bei Geräten zur Unterstützung einer Herzdruckmassage. Eine derartige Einrichtung wird bei der Reanimation verwendet und kombiniert häufig die Beatmungs- mit der Reanimationsfunktion. Es wird zum einen eine Beatmung und/oder Zuführung von Sauerstoff durchgeführt. Dem Helfer wird darüber hinaus eine Taktung für die Durchführung der Herzdruckmassage vorgegeben. Über die Anzeige wird zum einen die Taktung und zum anderen auch die Intensität des erforderlichen Druckes auf den Brustkorb vorgegeben. Bei entsprechenden Reanimationen liegen die erforderlichen Kompressionen in einem Bereich von 100 bis 120 Kompressionen pro Minute.

In den Zeichnungen sind Ausführungsbeispiele der Erfindung dargestellt. Es zeigen:
- Fig. 1: beispielhaft zwei Displayanzeigen eines exemplarisch gewählten Standes der Technik,
- Fig. 2: ein erstes Ausführungsbeispiel der erfindungsgemäßen Anzeige (100) mit Informationsgehalten bezüglich Parametern, Einstellwerten oder Messwerten und/oder deren Ausprägung in diskreter und/oder kontinuierlicher, alternierender, asymptotischer Weise und
- Fig. 3: ein beispielhaftes Interagieren der verschiedenen Geräte und/oder Vorrichtungen bei der Reanimation eines Patienten.

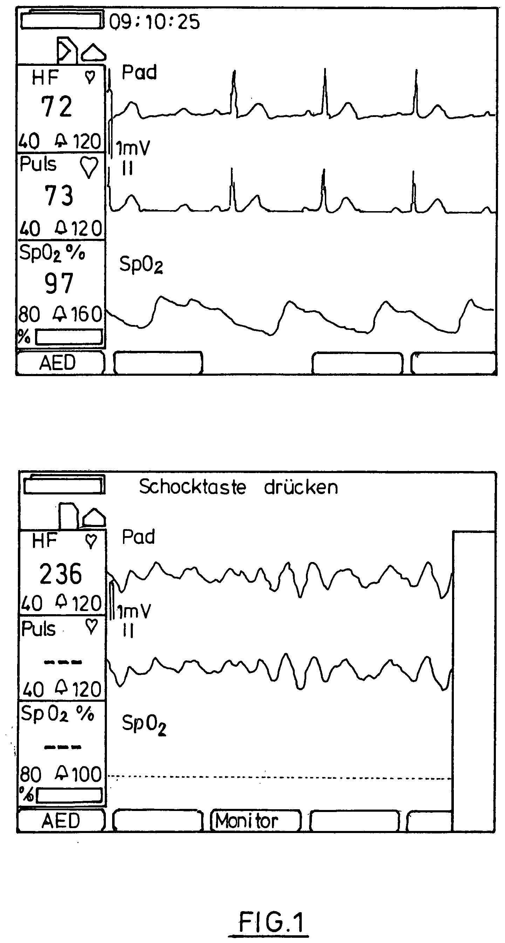

Figur 1 zeigt zwei Beispiele für Displayanzeigen umfassend jeweils eine Mehrzahl von Messwertfeldern zur Darstellung verschiedener Messwerte in Form von numerischen Werten und Graphen.

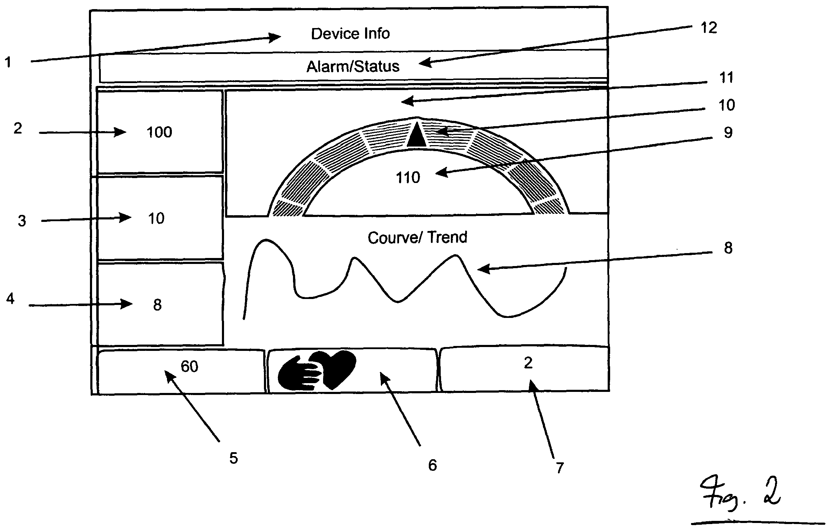

In Figur 2 ist die erfindungsgemäße Anzeige (100) in einer ersten möglichen Ausgestaltung abgebildet. Neben den optionalen Anzeigekomponenten (1) bis (8) und (12) ist wenigstens ein Anzeigebereich (11) tachometerartig ausgestaltet. Der tachometerartige Anzeigebereich (11) ist dabei durch eine Nadel (10) gebildet, die relativ zu einem Feld aus Werten oder Wertebereichen lageveränderlich enthalten ist und mit seiner Momentanposition den Informationsgehalt des vorliegenden Wertes anzeigt. Der anzuzeigende Wert ist ein Messwert (9), der zusätzlich zur angezeigten Information des Nadel (10) numerisch und in optisch erfassbarer Form dargestellt ist.

Die Bewegungskinematik der Nadel (10) oder dem tachometerartigen Anzeigebereich (11) mit einem Feld aus Werten zur Realisierung der relativen Lageveränderung zueinander über kann in Analogie zu Rundinstrumenten klassischer Tachometer in rotativer Weise erfolgen. Auch vorgesehen sind davon abweichende Bewegungsbahnen beispielsweise durch Freiformspuren, Linearbewegungen, elliptischen Kinematiken oder Wegstrecken entlang stochastisch regelloser Bahnen. Denkbar ist auch, dass die Nadel (10) ortsfest und das Feld aus Werten einer der genannten Bewegungskinematiken folgt. Gleiches gilt für kombinierte Bewegungskinematiken realisiert durch die Nadel (10) und dem Anzeigebereich (11).

Das in Figur 2 gezeigte Ausführungsbeispiel sieht für die Nadel (10) eine rotative Bewegungskinematik mit möglichen Momentanpositionen über beziehungsweise innerhalb von halbrund angeordneten und von der Nadel (10) überstreichbaren Wertebereichen vor.

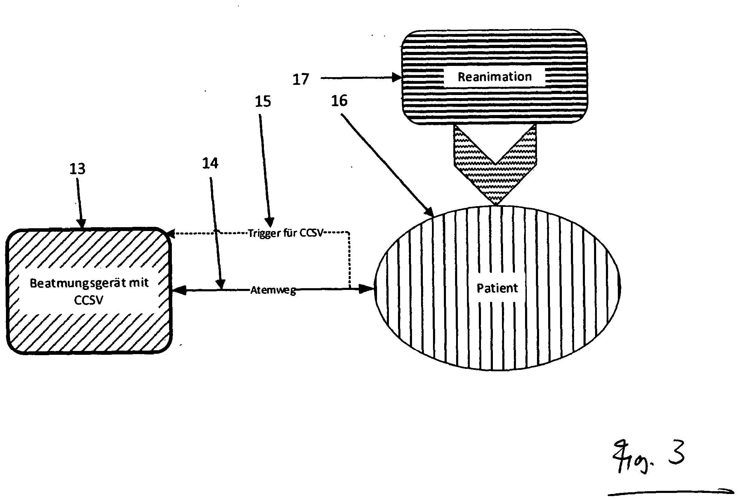

In Figur 3 ist ein beispielhaftes Interagieren der verschiedenen Geräte und/oder Vorrichtungen bei der Reanimation eines Patienten abgebildet. Die Reanimation beziehungsweise Thoraxkompression (17) dient zur Wiederbelebung eines Patienten (16) und wirkt einem Atem- und Kreislaufstillstand entgegen. Die Thoraxkompression (17) ist erfingungsgemäß, und wie in Figur 3 gezeigt mit einem Beatmungsgerät oder Notfallbeatmungsgerät (13) kombiniert. Dazu wird der Patient (16) mittels eines Beatmungsschlauchsystems (14) mit dem Beatmungsgerät oder Notfallbeatmungsgerät (13) gekoppelt. Das Beatmungsschlauchsystem (14) kann optional eine Atemwegssicherung aufweisen.

Offenbarungsgemäß kann das Beatmungsgerät oder Notfallbeatmungsgerät (13) das Beatmungsmuster CCSV (Chest Compression Synchronised Ventilation) realisieren. Dieses Beatmungsmuster unterstützt die simultane Insufflation mit jeder Thoraxkompression mit vorzugsweise folgenden Ventilationsparametern: Tinsp= 0.2 s, Pinsp= 60 (+-10%) mbar, f = 100/min, PEEP= 0 mbar, Pmax=65 mbar, FiO2=1,0. Ein atmungsabhängiges Triggersignal (15) wird dem Beatmungsgerät, Notfallbeatmungsgerät (13) zur Verfügung gestellt und kann insbesondere ein innerhalb des tachometerartigen Anzeigebereiches (11) der Anzeige (100) darzustellender Messwert sein.

## Patentansprüche

1. Medizintechnische Vorrichtung (17) zur Unterstützung einer Herzdruckmassage bei einer Reanimation mit einer Anzeige (100) zur Ausgabe von Informationsgehalten wenigstens eines Messwertes (9) innerhalb wenigstens eines Anzeigenbereiches, bei der der wenigstens eine Anzeigenbereich als tachometerartige Anzeige (11) ausgebildet ist, wobei die medizintechnische Vorrichtung (17) ein Notfallbeatmungsgerät (13) aufweist und wobei die Anzeige (100) als ein Display ausgebildet ist, welches eine Taktung einer Thoraxkompression als auch die Intensität des erforderlichen Druckes auf den Brustkorb visualisiert, wobei die tachometerartige Anzeige (11) innerhalb wenigstens eines Anzeigenbereiches durch wenigstens eine Nadel (10) und wenigstens einem Feld aus Werten gebildet ist und wobei die Lageänderung der wenigstens einen Nadel (10) relativ zu dem Feld durch die Bewegung der Nadel erfolgt und mit seiner Momentanposition den Informationsgehalt eines vorliegenden Messwerts (9) anzeigt, wobei die Bewegungskinematik der Nadel zur relativen Lageänderung einer rotativen Bewegungsbahn gehorcht, wobei das Feld aus Werten in halbrunder Weise innerhalb des Anzeigenbereiches angeordnet ist, wobei die Ausgabe von Informationsgehalten wenigstens eines Messwertes (9) medizintechnischer Vorrichtungen (13) additiv durch die Visualisierung des numerischen Wertes innerhalb der tachometerartigen Anzeige (11) realisiert ist, wobei der Informationsgehalt des wenigstens einen Messwertes (9) in unterschiedlichen Farbgebungen angezeigt ist und wobei die Farbgebung des wenigstens einen Messwertes (9) in eine rote Farbe wechselt, wenn der Informationsgehalt eine Grenzwertüberschreitung und/oder ein Alarm ist und wobei zusätzlich unter- oder oberhalb der tachometerartigen Anzeige (11) eine Kurve oder Trenddarstellungen dargestellt ist, wobei die Vorrichtung eine Beatmungsfunktion derart mit einer Reanimationsfunktion kombiniert, dass einem Benutzer sowohl eine Taktung für die Herzdruckmassage als auch die Intensität für einen Druck auf einen Brustkorb vorgegeben sind.

2. Medizintechnische Vorrichtung (17) nach Anspruch 1, **dadurch gekennzeichnet, dass** die Anzeige (100) ein Display oder Bildschirm oder bistabiles Display oder E-Paper-Display oder ein Touchscreen ist.

3. Medizintechnische Vorrichtung (17) nach Anspruch 1, **dadurch gekennzeichnet, dass** der Informationsgehalt des wenigstens einen Messwertes (9) als diskrete oder fortlaufende oder alternierende oder intermittierende biologische oder physikalische Größe angezeigt ist.

4. Medizintechnische Vorrichtung (17) nach Anspruch 1, **dadurch gekennzeichnet, dass** der wenigstens eine Messwert (9) eine Triggerfrequenz und/oder eine Soll- oder Ist-Kompressionstaktung und/oder eine Pulsfrequenz ist.

## Claims

1. Medical apparatus (17) for supporting cardiac massage during resuscitation, having a display (100) for outputting information contents of at least one measured value (9) within at least one display region, in which the at least one display region is in the form of a tachometer-type display (11), wherein the medical apparatus (17) has an emergency ventilator (13) and wherein the display (100) is embodied in the form of a display unit that visualizes a rate of a chest compression and also the intensity of the required pressure exerted on the chest, wherein the tachometer-type display (11) within at least one display region is formed by at least one needle (10) and at least one field of values and wherein the changing position of the at least one needle (10) relative to the field is caused by the movement of the needle and displays with its instantaneous position the information content of an available measured value (9), wherein the movement kinematics of the needle for the relative change in position follows a rotational movement path, wherein the field of values is arranged in a semicircular manner within the display region, wherein the output of information contents of at least one measured value (9) of medical apparatuses (13) is realized additively by the visualisation of the numeric value within the tachometer-type display (11), wherein the information content of the at least one measured value (9) is displayed in different colours and wherein the colour of the at least one measured value (9) changes to red if the information content indicates a limit value being exceeded and/or is an alert and wherein additionally a curve or trend charts is/are presented below or above the tachometer-type display (11), wherein the apparatus combines a ventilation function with a resuscitation function in a manner such that a user is provided both with a rate for the cardiac massage and also with the intensity of a chest compression.

2. Medical apparatus (17) according to Claim 1, **characterized in that** the display (100) is a display unit or a screen or a bistable display unit or an e-paper display unit or a touchscreen.

3. Medical apparatus (17) according to Claim 1, **characterized in that** the information content of the at least one measured value (9) is displayed as a discrete or continuous or alternating or intermittent biological or physical variable.

4. Medical apparatus (17) according to Claim 1, **characterized in that** the at least one measured value (9) is a trigger frequency and/or a predetermined or actual compression rate and/or a pulse frequency.

## Revendications

1. Dispositif médical (17) destiné à assister un massage cardiaque lors d'une réanimation, ledit dispositif médical comprenant un affichage (100) destiné à délivrer le contenu informatif d'au moins une valeur de mesure (9) à l'intérieur d'au moins une zone d'affichage, l'au moins une zone d'affichage étant conçue comme un affichage de type tachymètre (11), le dispositif médical (17) comportant un respirateur d'urgence (13) et l'affichage (100) étant conçu comme un affichage qui visualise le cadencement d'une compression thoracique ainsi que l'intensité de la pression requise exercée sur la cage thoracique, l'affichage de type tachymètre (11) dans au moins une zone d'affichage étant formé par au moins une aiguille (10) et au moins une plage de valeurs et le changement de position d'au moins une aiguille (10) par rapport à la plage étant effectué par le mouvement de l'aiguille et sa position instantanée indiquant le contenu informatif d'une valeur de mesure présente (9), la cinématique de mouvement de l'aiguille obéissant au changement de position relatif d'une trajectoire en rotation, la plage de valeurs étant disposée de manière semi-circulaire à l'intérieur de la zone d'affichage, la délivrance du contenu informatif d'au moins une valeur de mesure (9) de dispositifs médicaux (13) étant réalisée de manière additive par la visualisation de la valeur numérique à l'intérieur de l'affichage de type tachymètre (11), le contenu informatif de l'au moins une valeur de mesure (9) étant affiché dans différentes colorations et la coloration de l'au moins une valeur de mesure (9) passant à une couleur rouge si le contenu informatif est un dépassement de valeur limite et/ou une alarme et une courbe ou des représentations de tendance étant en outre représentées au-dessous ou au-dessus de l'affichage de type tachymètre (11), le dispositif combinant une fonction de respiration à une fonction de réanimation de manière à ce que aussi bien le cadencement du massage cardiaque et l'intensité d'une pression exercée sur une cage thoracique soient spécifiés pour un utilisateur.

2. Dispositif médical (17) selon la revendication 1, **caractérisé en ce que** l'affichage (100) est un afficheur ou un écran ou un afficheur bistable ou un afficheur e-paper ou un écran tactile.

3. Dispositif médical (17) selon la revendication 1, **caractérisé en ce que** le contenu informatif de l'au moins une valeur de mesure (9) est affiché sous la forme d'une grandeur biologique ou physique discrète ou continue ou alternée ou intermittente.

4. Dispositif médical (17) selon l'une des revendications précédentes, **caractérisé en ce que** l'au moins une valeur de mesure (9) est une fréquence de déclenchement et/ou un cadencement de compression cible ou réel et/ou une fréquence d'impulsion.
